# FASCICULE DE BREVET EUROPEEN

(11) **EP 0 828 740 B1**
(45) Date de publication et mention de la délivrance du brevet: **05.09.2001**
(21) Numéro de dépôt: 96917555.3
(22) Date de dépôt: 28.05.1996
(51) Int. Cl.: C07D 471/08

(54) **DERIVES DU 2-AZABICYCLO[2.2.1]HEPTANE, LEUR PREPARATION ET LEUR APPLICATION**
2-AZABICYCLO[2.2.1]HEPTAN-DERIVATE, IHRE HERSTELLUNG UND IHRE ANWENDUNG
2-AZABICYCLO[2.2.1]HEPTANE DERIVATIVES, PREPARATION AND APPLICATION THEREOF

(30) Priorité: 30.05.1995 FR 9506353
(43) Date de publication de la demande: 18.03.1998
(73) Titulaire: Aventis Pharma S.A., 92160 Antony (FR)
(72) Inventeur: LARGEAU, Denis, F-69440 Taluyers (FR); LEON, Patrick, F-69160 Tassin-la-Demi-Lune (FR)
(86) Numéro de dépôt international: FR9600793
(87) Numéro de publication internationale: WO9638447

(56) Documents cités:
- CHEMICAL AND PHARMACEUTICAL BULLETIN, vol. 39, no. 5, 1991, TOKYO JP, pages 1112-1122, XP002014503 N. KATAGIRI ET AL.:
- TETRAHEDRON LETTERS, vol. 30, no. 13, 1989, OXFORD GB, pages 1645-1648, XP002014504 N. KATAGIRI ET AL.:
- TETRAHEDRON LETTERS, vol. 30, no. 41, 1989, OXFORD GB, pages 5543-5546, XP002014505 J. CHEN ET AL.:
- JOURNAL OF ORGANIC CHEMISTRY, vol. 46, 1981, EASTON US, pages 3268-3272, XP002014506 B.L. KAM ET AL.:
- TETRAHEDRON LETTERS, vol. 22, no. 25, 1981, OXFORD GB, pages 2331-2332, XP002014507 R.C. CERMAK ET AL.:
- J. Am. Chem. Soc. (1985), vol.107, 1768-1769

## Description

La présente invention concerne de nouveaux dérivés du 2-azabicyclo [2.2.1]heptane 1R de formule générale: 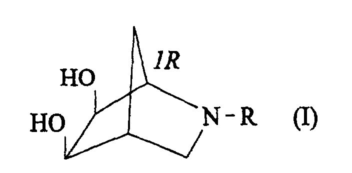 leur préparation et leur application.

Dans la formule générale (I), R représente un radical atome d'hydrogène ou un radical de formule générale: 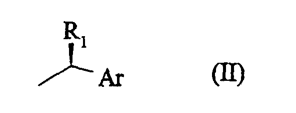 dans laquelle R₁ représente un radical alcoyle contenant 1 à 4 atomes de carbone et Ar représente un radical phényle ou α- ou β-naphtylé éventuellement substitué par un ou plusieurs atomes ou radicaux identiques ou différents choisis parmi les atomes d'halogène et les radicaux alcoyles contenant 1 à 4 atomes de carbone, alcoxy contenant 1 à 4 atomes de carbone ou nitro.

De préférence, R₁ représente un radical méthyle ou éthyle et Ar représente un radical phényle éventuellement substitué par un ou plusieurs radicaux méthyle ou méthoxy.

Plus particulièrement encore, R₁ représente un radical méthyle et Ar représente un radical phényle.

Il est connu selon l'article de Chen J., Grim M., Rock C., Chan K. paru dans Tetrahedron Letters, vol. 30, No. 41, pages 5543-5546 (1989) de résoudre la stéréochimie d'une azabicycloheptanone par ouverture de cette dernière au moyen d'une amine de façon à former un groupe carbamoyle qui est résolu au moyen de l'acide (+) dibenzyltartrique.

Il est encore connu d'après les articles de Katagiri N., Muto M., Kaneko C., paru dans Tetrahedron Letters, voL 30, No.13, pages 1645-1648 (1989) et de Katagiri N., Muto M., Nomura M., Higashikawa T., Kaneko C. Paru dans Chem. Pharm. Bull., vol. 39(5), pages 1112-1122 (1991) un moyen de résoudre là encore des azabicycloheptanones en passant intermédiairement par des groupes carbamoyles.

Selon l'invention, les produits de formule générale (I) dans laquelles R représente un radical de formule générale (II) peuvent être obtenus par bis-hydroxylation d'un produit de formule générale : 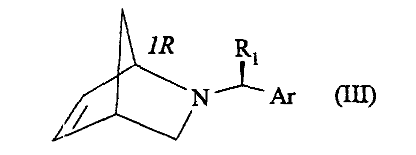 dans laquelle R₁ et Ar sont définis comme précédemment,

Généralement, la bis-hydroxylation est effectuée en opérant dans les conditions décrites par V. VanRheenen et coll., Tetrahedron Letters, 23, 1973-1976 (1976). Plus particulièrement, l'oxydation peut être réalisée au moyen de permanganate de potassium ou de tétroxyde d'osmium en opérant en présence de N-méthyl morpholine-oxyde ou de triéthylamine-oxyde ou de ferricyanure de potassium (K₃FeCN₆). Généralement, on opère dans un milieu hydro-organique tel qu'un mélange eau-tert.butanol ou eau-acétone.

D'une manière générale, l'oxydant doit être choisi de telle manière qu'il ne se forme que le dérivé 5,6-dihydroxy sous forme exo.

Le produit de formule générale (III) peut être obtenu par réaction de Diels-Alder entre une amine homochirale de formule générale: 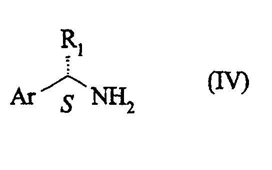 dans laquelle R₁ et Ar sont définis comme précédemment, sous forme de sel, de préférence avec un acide minéral tel que l'acide chlorhydrique, le formaldéhyde et le cyclopentadiène en opérant dans les conditions décrites par S.D. Larsen et P.A. Grieco, J. Amer. Chem. Soc., 107, 1768-1769 (1985).

La mise en oeuvre du procédé conduit, à partir d'une amine homochirale de forme S, à un mélange de 2 diastéréoisomères qui, réagissant de la même manière dans l'étape ultérieure de bis-hydroxylation, ne doivent pas nécessairement être séparés.

Selon l'invention, le produit de formule générale (I) dans laquelle R représente un atome d'hydrogène peut être obtenu par hydrogénolyse d'un produit de formule générale (I) dans laquelle R représente un radical de formule générale (II) au moyen d'hydrogène en présence d'un catalyseur tel que le palladium sur charbon en opérant dans un solvant organique tel qu'un alcool comme le méthanol.

L'isomère 1R de formule générale (I) dans laquelle R représente un radical de formule générale (II) peut aussi être isolé d'un mélange des produits de formule générale (I) et (I') 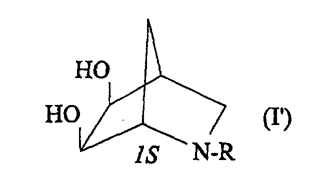 par cristallisation diastéréosélective avec un acide organique optiquement actif dans un solvant organique approprié. Il est particulièrement avantageux d'utiliser l'acide L-diméthoxysuccinique dans un alcoool aliphatique tel que l'isopropanol.

Les nouveaux produits de formule générale (I) sont particulièrement utiles pour préparer les produits qui font l'objet du brevet américain US 5,364,862 et qui sont des agents actifs dans le traitement des maladies cardiovasculaires telles que l'hypertension et l'ischémie myocardiale.

D'un intérêt tout particulier est le [1-S-[1α,2β,3β,4α(S*)]]-4-[7-[[2-(3-chloro-2-thiényl)-1-éthyléthyl]amino]-3H-imidazo[4,5-b]pyridin-3-yl]-N-éthyl-2,3-dihydroxycyclopentane-carboxamide de formule :

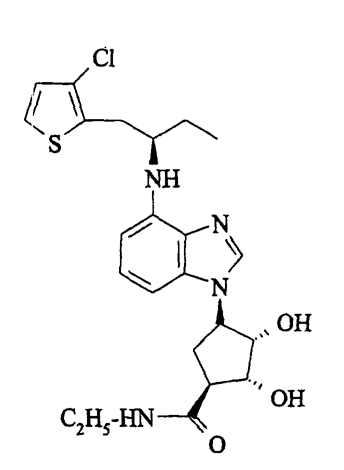

Les produits de formule générale (I) sont particulièrement utiles pour préparer le carbosucre de formule générale : 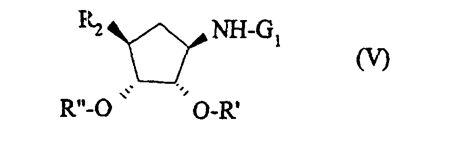 dans laquelle R₂ représente un radical carboxy, alcoxycarbonyle dont la partie alcoyle contient 1 à 4 atomes de carbone, N-alcoylaminocarbonyle dont la partie alcoyle contient 1 à 4 atomes de carbone ou hydroxyméthyle ou alcoxyméthyle, et R' et R", identiques ou différents, représentent un atome d'hydrogène ou un reste d'acide organique aliphatique contenant 2 à 4 atomes de carbone, tel qu'un radical acétyle ou propionyle, ou un reste benzoyle ou bien R' et R" forment ensemble un radical méthylène dont l'atome de carbone est éventuellement substitué par un ou plusieurs radicaux, identiques ou différents choisis parmi les radicaux alcoyles contenant 1 à 4 atomes de carbone pouvant former ensemble un radical alicyclique contenant 5 ou 6 atomes de carbone, ou les radicaux phényles, et G₁ représente un atome d'hydrogène ou un groupement protecteur G₂ de la fonction amino. Plus particulièrement, R₂ représente un radical éthylaminocarbonyle ou hydroxyméthyle et R' et R" forment ensemble un radical isopropylidène.

Le carbosucre de formule générale (V) constitue un des éléments de la structure des produits revendiqués dans le brevet américain US 5,364,862.

La préparation du carbosucre de formule générale (V) à partir du produit de formule générale (I) peut être réalisée de la manière suivante.

Les fonctions hydroxy du produit de formule générale (I) dans laquelle R représente un atome d'hydrogène ou un radical de formule générale (II) peuvent être protégées sous forme d'ester ou d'acétal pour donner un produit de formule générale : 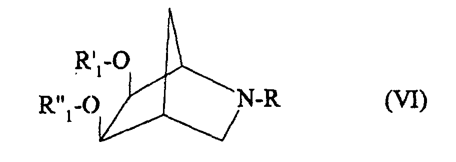 dans laquelle R représente un atome d'hydrogène ou un radical de formule générale (II) et R'₁ et R"₁, identiques ou différents, représentent un reste d'acide organique aliphatique contenant 2 à 4 atomes de carbone, tel qu'un radical acétyle ou propionyle, ou un reste benzoyle ou bien R'₁ et R"₁ forment ensemble un radical méthylène dont l'atome de carbone est éventuellement substitué par un ou plusieurs radicaux, identiques ou différents choisis parmi les radicaux alcoyles contenant 1 à 4 atomes de carbone pouvant former ensemble un radical alicyclique contenant 5 ou 6 atomes de carbone, ou les radicaux phényles

Généralement, la protection des radicaux hydroxy s'effectue dans les conditions habituelles d'estérification ou d'acétalisation par exemple par action de l'acide acétique ou propionique en présence d'acide p.toluène sulfonique dans un solvant organique tel qu'un hydrocarbure aromatique comme le benzène ou le toluène en séparant l'eau au fur et à mesure de sa formation ou par action d'un aldéhyde ou d'une cétone éventuellement sous forme d'acétal en présence d'un acide tel que l'acide trifluoroacétique dans un solvant organique tel qu'un hydrocarbure aromatique comme le benzène ou le toluène, à une température comprise entre 50°C et la température de reflux du mélange réactionnel.

Le produit de formule générale (VI) dans laquelle R représente un radical de formule générale (II) peut être transformé en produit de formule générale (VI) dans laquelle R représente un atome d'hydrogène par hydrogénolyse.

Généralement, l'hydrogénolyse est effectuée au moyen d'hydrogène, éventuellement sous pression, en présence d'un catalyseur tel que le palladium sur charbon dans un solvant organique tel qu'un alcool comme le méthanol, l'éthanol ou l'isopropanol à une température comprise entre 0 et 50°C.

Le produit de formule générale (VI), qui est un produit nouveau, constitue un autre objet de la présente invention.

Le produit de formule générale (VI) dans laquelle R représente un atome d'hydrogène peut être transformé en produit de formule générale : 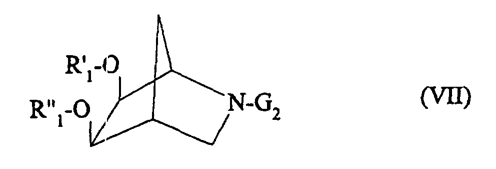 dans laquelle R'₁ et R"₁ sont définis comme précédemment et G₂ représente un groupement protecteur de la fonction amino par action d'un réactif convenable permettant l'introduction sélective d'un groupement protecteur.

Les groupements protecteurs sont choisis parmi ceux qui peuvent être ultérieurement éliminés sélectivement. Parmi les groupements protecteurs qui conviennent particulièrement bien peuvent être cités les radicaux chloroacétyle, méthoxyméthyle, trichloro-2,2,2 éthoxycarbonyle, t.butyle, benzyle, p.nitrobenzyle, p.méthoxybenzyle, diphénylméthyle, trialcoylsilyle, allyloxycarbonyle, benzyloxycarbonyle, dans lequel le noyau phényle est éventuellement substitué par un atome d'halogène ou par un radical alcoyle contenant 1 à 4 atomes de carbone ou alcoyloxy contenant 1 à 4 atomes de carbone, ou t.butoxycarbonyle. Parmi les autres groupements protecteurs qui conviennent particulièrement bien peuvent être cités ceux qui sont décrits par T.W. Greene et P.G.M. Wuts, "Protecting Groups in Organic Synthesis", Chapître 7,2ème édition, John Wiley & Sons (1991).

D'un intérêt tout particulier est le groupement t.butoxycarbonyle.

Le produit de formule générale (VII) dans laquelle G₂ représente un radical t.butoxycarbonyle peut être obtenu directement à partir d'un produit de formule générale (VI) dans laquelle R représente un radical de formule générale (II) par hydrogénolyse et tert.butoxycarbonylation simultanées.

Généralement, la réaction est mise en oeuvre en faisant réagir simultanément l'hydrogène en présence d'un catalyseur tel que le palladium sur charbon et le dicarbonate de di-t.butyle sur un produit de formule générale (VI) en opérant dans un solvant organique tel qu'un alcool comme le méthanol, l'éthanol ou l'isopropanol à une température comprise entre 0 et 50°C.

Le produit de formule générale (VII) est un produit nouveau qui constitue un autre objet de la présente invention.

Le produit de formule générale (VII) est ensuite oxydé en produit de formule générale : 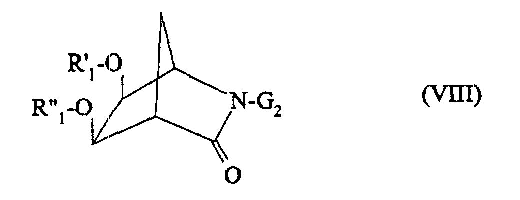 dans laquelle R'₁, R"₁ et G₂ sont définis comme précédemment.

Généralement, l'oxydation est réalisée au moyen d'oxyde de ruthénium (RuO₄) éventuellement généré in situ à partir d'un précurseur tel que RuO₂ ou RuCl₃ en présence d'un oxydant choisi parmi un periodate tel que le periodate de sodium, un hypochlorite tel que l'hypochlorite ou l'hypobromite de sodium ou un bromate tel que le bromate de sodium ou un oxyde d'amine tertiaire organique tel que la N-méthylmorpholine-oxyde ou la triéthylamine-oxyde en opérant dans l'eau ou dans un milieu hydro-organique homogène ou hétérogène tel qu'un mélange eau-acétate d'éthyle.

L'oxydation peut aussi être réalisée au moyen d'hypochlorite de sodium seul (eau de Javel) ou au moyen de permanganate de potassium ou au moyen de tungstate de sodium en présence d'un oxydant tel que l'hypochlorite de sodium, l'eau oxygénée ou un hydroperoxyde d'alcoyle.

Le produit de formule générale (VIII) peut aussi être obtenu par oxydation d'un produit de formule générale (VI) dans laquelle R représente un atome d'hydrogène dans les conditions décrites ci-dessus, suivie de la protection de l'atome d'azote du lactame obtenu de formule générale : 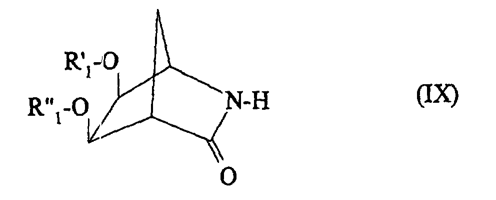 dans laquelle R'₁ et R"₁ sont définis comme précédemment, par un groupement protecteur tel que défini précédemment.

Le produit de formule générale (VIII) peut être transformé en produit de formule générale (V) dans des conditions appropriées à la nature du substituant R₂ qui doit être introduit.

Le produit de formule générale (V) dans laquelle R₂ représente un radical carboxy peut être obtenu par action d'une base minérale telle que la soude sur le produit de formule générale (VIII), suivie du remplacement du groupement protecteur G₂ par un atome d'hydrogène et éventuellement des radicaux R'₁ et R"₁ par des atomes d'hydrogène.

Le produit de formule générale (V) dans laquelle R₂ représente un radical carboxy peut être obtenu par remplacement du groupement protecteur G₂ du produit de formule générale (VIII) par un atome d'hydrogène suivi de l'action d'une d'une base minérale telle que la soude, et éventuellement du remplacement des radicaux R'₁ et R" par des atomes d'hydrogène.

Le produit de formule générale (V) dans laquelle R₂ représente un radical alcoxycarbonyle dont la partie alcoyle contient 1 à 4 atomes de carbone peut être obtenu par action d'un alcoolate de métal alcalin sur le produit de formule générale (VIII), suivie du remplacement du groupement protecteur G₂ par un atome d'hydrogène et éventuellement des radicaux R'₁ et R"₁ par des atomes d'hydrogène.

Le produit de formule générale (V) dans laquelle R₂ représente un radical alcoxycarbonyle dont la partie alcoyle contient 1 à 4 atomes de carbone peut être obtenu par remplacement du groupement protecteur G₂ du produit de formule générale (VIII) par un atome d'hydrogène suivi de l'action d'un alcoolate de métal alcalin, et éventuellement du remplacement des radicaux R'₁ et R"₁ par des atomes d'hydrogène.

Le produit de formule générale (V) dans laquelle R₂ représente un radical N-alcoylaminocarbonyle dont la partie alcoyle contient 1 à 4 atomes de carbone peut être obtenu par action d'une alcoylamine sur le produit de formule générale (VIII), suivi remplacement du groupement protecteur G₂ par un atome d'hydrogène, et éventuellement du remplacement des radicaux R'₁ et R"₁ par des atomes d'hydrogène.

Le produit de formule générale (V) dans laquelle R₂ représente un radical N-alcoylaminocarbonyle dont la partie alcoyle contient 1 à 4 atomes de carbone peut être obtenu par remplacement du groupement protecteur G₂ du produit de formule générale (VIII) par un atome d'hydrogène, suivi de l'action d'une alcoylamine, et éventuellement du remplacement des radicaux R'₁ et R"₁ par des atomes d'hydrogène.

Le produit de formule générale (V) dans laquelle R₂ représente un radical hydroxyméthyle peut être obtenu par action d'un agent réducteur tel qu'un borohydrure comme le borohydrure de sodium ou de potassium sur le produit de formule générale (VIII), suivie du remplacement du groupement protecteur G₂ par un atome d'hydrogène et éventuellement des radicaux R'₁ et R"₁ par des atomes d'hydrogène.

Le produit de formule générale (V) dans laquelle R₂ représente un radical hydroxyméthyle peut être obtenu par remplacement du groupement protecteur G₂ du produit de formule générale (VIII) par un atome d'hydrogène suivi de l'action d'un agent réducteur tel qu'un borohydrure comme le borohydrure de sodium ou de potassium, et du remplacement éventuel des radicaux R'₁ et R"₁ par des atomes d'hydrogène.

Le produit de formule générale (V) peut être utilisé dans les conditions décrites dans le brevet américain US 5,364,862 pour obtenir les produits thérapeutiquement actifs.

Les exemples suivants illustrent la présente invention.

### EXEMPLE 1

Dans un ballon tricot de 250 cm3 muni d'un réfrigérant et d'un système d'agitation, on introduit, sous atmosphère d'argon, une solution de 20 g de α-S-méthylbenzylamine (165 mmoles) dans 60 cm3 d'eau dont le pH est ajusté à 6,10 par addition de 17 cm3 d'acide chlorhydrique à 36 % (p/v). Après refroidissement à 5°C, on ajoute 20 cm3 d'une solution aqueuse de formaldéhyde à 37 % (p/v). On agite pendant 5 minutes à 5°C puis on ajoute 21,8 g de cyclopentadiène (330 mmoles). On agite pendant 16 heures entre -5 et 0°C. La phase aqueuse est séparée par décantation puis lavée par 50 cm3 de pentane. On neutralise à pH = 8,0 par addition de soude concentrée. On extrait alors avec 2 fois 70 cm3 d'acétate d'éthyle. La phase aqueuse est amenée à pH = 11 par addition de soude concentrée puis on extrait avec 2 fois 70 cm3 d'acétate d'éthyle. Les phases organiques sont réunies puis lavées avec 2 fois 50 cm3 d'eau puis séchées sur sulfate de sodium. Après filtration et concentration à sec sous pression réduite, on obtient 33,10 g de 2-(a-S-méthylbenzyl)-2-azabicyclo [2,2,1]hept-5-ene sous forme d'une huile légèrement jaune.

Dans un ballon tricol de 500 cm3 muni d'un réfrigérant et d'un système d'agitation, contenant une solution de 20 g de 2-(α-S-méthylbenzyl)-2-azabicyclo [2,2,1]hept-5-ene (75,34 mmoles) dans 220 cm3 de tert-butanol, on ajoute, à une température voisine de 25°C, 12 g de N-méthylmorpholine-oxyde dans 32 cm3 d'eau puis, lentement, 6,3 cm3 d'une solution à 2,5 % (p/v) de tétroxyde d'osmium (OsO₄) dans le tert-butanol. On agite pendant 2 heures à une température voisine de 20°C puis pendant 3 heures à 65°C. Après évaporation du tert-butanol sous pression réduite, le résidu est repris par 350 cm3 d'isopropanoL Après concentration à sec sous pression réduite, on obtient 24 g de cis-5,6-dihydroxy-2-(a-S-methylbenzyl)-2-azabicyclo [2,2,1]heptane sous forme d'une huile. On obtient, par cristallisation dans le cyclohexane, 14 g de 5R,6S-dihydroxy-2-(α-S-methylbenzyl)-2-azabicyclo[2,2,1] heptane dont la pureté isomérique est supérieure à 95 %.

Le spectre de R.M.N., déterminé dans le chloroforme deutéré, montre les déplacements chimiques (δ) suivants : 1,21 (3H, d) ; 1,38 (1H, d) ; 1,59 (1H, d) ; 2,22 (2H, m) ; 2,45 (1H, dd) ; 2,95 (1H, s) ; 3,39 (1H, q) ; 3,78 (1H, d) ; 3,90 (1H, d) ; 7,28 (5H, m).

### EXEMPLE 2

Dans un ballon tricol de 500 cm3, muni d'un réfrigérant et d'un système d'agitation, contenant une solution de 18,4 g de 5R,6S-dihydroxy-2-(α-S-methylbenzyl)-2-azabicyclo[2,2,1]heptane (76 mmoles) dans 130 cm3 de toluène, on ajoute 31,7 g de 2,2-diméthoxypropane (304 mmoles) puis, lentement, 13 g d'acide trifluoroacétique (114 mmoles). On chauffe pendant 4 heures 10 minutes à 65°C. Après refroidissement à 30°C et concentration à l'évaporateur rotatif pour éliminer le toluène, l'excès de 2,2-diméthoxypropane et partiellement l'acide trifluoroacétique, le mélange réactionnel est repris par du dichlorométhane puis est neutralisé par addition de 100 cm3 de soude 2N. Après décantation, séchage de la phase organique sur sulfate de sodium, filtration, traitement au noir décolorant (30 g) pendant 30 minutes au reflux du dichlorométhane, filtration sur clarcel ert concentration à sec sous pression réduite, on obtient 18,8 g de 5R,6S-isopropylidènedioxy-2-(α-S-méthylbenzyl)-2-azabicyclo[2,2,1]heptane dont la structure est confirmée par le spectre de R.M.N. du proton, qui, déterminé dans le chloroforme deutéré, montre les déplacements chimiques (8) suivants : 1,22 (3H,d) ; 1,23 (6H, s) ; 1,31 (1H, d) ; 1,57 (1H, d) ; 2,08 (1H, d) ; 2,34 (1H, s large) ; 2,45 (1H, dd) ; 3,06 (1H, s) ; 3,40 (1H, q) ; 4,09 (1H, d); 4,19 (1H, d); 7,26 (5H, m).

Dans un ballon tricol de 250 cm3 muni d'un système d'agitation, on introduit 0,5 g de palladium sur charbon à 5 % en poids, 5 g de 5R,6S-isopropylidènedioxy-2-(α-S-méthylbenzyl)-2-azabicyclo[2,2,1]heptane, 3,98 g de dicarbonate de di-tert.butyle et 36 cm3 de méthanoL L'appareil est purgé à l'argon puis à l'hydrogène puis mis sous atmosphère d'hydrogène à 25°C. On poursuit la réaction pendant 5 heures en réalisant une purge à l'hydrogène chaque quart d'heure afin d'éliminer le gaz carbonique formé.

Après filtration sur clarcel et concentration à sec sous pression réduite, on obtient 4,84 g de 5R,6S-isopropylidènedioxy-2-(tert.butoxycarbonyl)-2-azabicyclo [2,2,1]heptane dont la structure est confirmée par le spectre de R.M.N. qui, déterminé dans le diméthylsulfoxyde-d6, montre les déplacements chimiques (8) suivants : 1,16 (s, 3H) ; 1,28 (s, 3H) ; 1,32 (s, 1H) ; 1,34 (s,3H) ; 1,65 (d, 1H) ; 2,38 (m, 1H) ; 2,65 (d, 1H) ; 2,99 (m, 1H); 3,84 (m, 1H); 3,94 (d, 1H); 4,16 (d, 1H).

Dans un tube de 30 cm3, on introduit 270 mg de 5R,6S-isopropylidènedioxy-2-(tert.butoxycarbonyl)-2-azabicyclo[2,2,1]heptane (1 mmole) et 40 mg de RuO₂, H₂O (O,3 équivalent). On ajoute 10 cm3 d'acétate d'éthyle et 720 mg d'eau (40 équivalents). On ajoute ensuite 2,14 g de periodate de sodium (10 équivalents) et on scelle le tube hermétiquement. On agite pendant 16 heures à 50°C. Le mélange réactionnel est filtré sur clarcel puis on extrait par 2 fois 20 cm3 d'acétate d'éthyle. Les phases organiques sont séchées sur sulfate de sodium Après filtration et concentration à sec sous pression réduite, on obtient 245 mg d'un solide contenant 68 % de 5R,6S-isopropylidènedioxy-2-(tert.butoxycarbonyl)-2-azabicyclo[2,2,1] heptane-3-one et 32 % de produit de départ. La structure du produit obtenu est confirmée par le spectre de R.M.N. qui, déterminé dans le diméthylsulfoxyde d6, montre les déplacements chimiques (δ) suivants : 1,38 (9H, s) ; 1,23 (3H, s) ; 1,33 (3H, s) ; 1,85 (1H, d) ; 1,93 (1H, d) ; 2,69 (1H, s) ; 4,24 (1H, s) ; 4,41 (1H, d) ; 4,51 (1H, d).

### EXEMPLE 3

Dans un autoclave de 25 cm3, muni d'une agitation magnétique, on introduit 1,47 g de 5R,6S-isopropylidènedioxy-2-(tert.butoxycarbonyl)-2-azabicyclo[2,2,1] heptane-3-one en solution dans 10 cm3 de toluène anhydre, puis environ 0,7 cm3 d'éthylamine. On ferme l'autoclave que l'on chauffe à un température comprise entre 90 et 100°C pendant 21 heures. Après refroidissement, on évapore le toluène et on reprend par 10 cm3 de dichlorométhane et 10 cm3 d'eau. Après décantation, la phase organique est lavée par 10 cm3 d'eau. .Les couches aqueuses réunies sont lavées par 10 cm3 de dichlorométhane Les phases organiques réunies sont lavées par 10 cm3 d'une solution saturée de chlorure de sodium puis séchées sur sulfate de sodium. Après filtration et concentration à sec sous pression réduite, on obtient 1,58 g d'un produit contenant 95 % de 2R,3S,-isopropylidènedioxy-4-R-tert-butoxycarbonylamino-1-S-éthylaminocarbonylcyclopentane dont la structure est confirmée par le spectre R.M.N. qui, déterminé dans le diméthylsulfoxyde d6, montre les déplacements chimiques suivants : 0,95 (t, 3H) ; 1,14 (s, 3H) ; 1,31 (s, 12H) ; 1,55 (m, 1H) ; 2,11 (m, 1H) ; 2,64 (m, 1H) ; 3,00 (qi, 2H) ; 3,77 (m, 1H) ; 4,23 (m, 1H) ; 4,54 (m, 1H) ; 7,07 (d, 1H) ; 8,12 (t, 1H). Dans un ballon de 25 cm3, on introduit 1,22 g de 2R,3S-isopropylidènedioxy-4R-tert.butoxycarbonylamino-1S-éthylaminocarbonylcyclopentane et 10 cm3 de dichlorométhane. A une température voisine de 25°C, on ajoute, sous agitation magnétique, 0,85 g d'acide trifluoroacétique. Après 6 heures d'agitation et concentration à sec, on obtient 1,16 g de trifluoroacétate de 2R,3S-isopropylidènedioxy-4R-amino-lS-éthylaminocarbonylcyclopentane dont la structure est confirmée par le spectre de R.M.N. qui, déterminé dans le diméthylsulfoxyde d6, montre les déplacements chimiques suivants : 0,79 (t, 3H) ; 1,03 (s, 3H) ; 1,19 (s, 3H); 1,42 (m, 1H) ; 2,05 (m, 1H) ; 2,52 (m, 1H) ; 2,89 (qi, 2H) ; 3,04 (m, 1H) ; 4,16 (m, 1H).

### EXEMPLE 4

Une solution de 0,5 mmoles d'un mélange (78/22 en moles) de 5R,6S-dihydroxy-2-(a-S-methylbenzyl)-2-azabicyclo[2,2,1]heptane et de 5S,6R-dihydroxy-2-(α-S-methylbenzyl)-2-azabicyclo[2,2,1]heptane et de 0,5 mmole d'acide L-diméthoxysuccinique dans 1 cm3 d'isopropanol est agitée pendant 24 heures à une température variant de 25°C au départ à 5°C. Les cristaux obtenus sont séparés par filtration et séchés. On obtient ainsi 110 mg de 5R,6S-dihydroxy-2-(a-S-methyl-benzyl)-2-azabicyclo[2,2,1]heptane avec un excès énantiomérique de 97 %.

Le mélange (78/22 en moles) de 5R,6S-dihydroxy-2-(α-S-methylbenzyl)-2-azabicyclo[2,2,1]heptane et de 5S,6R-dihydroxy-2-(α-S-methylbenzyl)-2-azabicyclo [2,2,1]heptane peut être obtenu de la manière suivante :

Dans un ballon tricol de 250 cm3, muni d'un réfrigérant et d'un système d'agitation, contenant une solution de 7 g de 2-(a-S-méthylbenzyl)-2-azabicyclo [2,2,1]hept-5-ene (35 mmoles) dans 70 cm3 de tert-butanol, on ajoute, à une température voisine de 25°C, 4,12 g de N-méthylmorpholine-oxyde dans 11 cm3 d'eau puis, lentement, 360 µl d'une solution à 2,5 % (p/v) de tétroxyde d'osmium (OsO₄) dans le tert-butanoL On agite pendant 1 heure à une température voisine de 20°C puis pendant 4 heures à 65°C. Après évaporation du tert-butanol sous pression réduite, le résidu est repris par 150 cm3 d'isopropanoL Après concentration à sec sous pression réduite, on obtient 8,27 g de d'un produit dont le spectre de R.M.N. du proton montre qu'il est constitué d'un mélange (78/22 en moles) de 5R,6S-dihydroxy-2-(α-S-methylbenzyl)-2-azabicyclo[2,2,1]heptane et de 5S,6R-dihydroxy-2-(α-S-methyl-benzyl)-2-azabicyclo[2,2,1]heptane.

### EXEMPLE 5

Dans un tube 'Berghoff, on introduit 568 mg de 5R,6S-isopropylidènedioxy-2-(tert.butoxycarbonyl)-2-azabicyclo[2,2,1] heptane-3-one et 10 cm3 d'une solution aqueuse d'éthylamine à 70 % (en poids). On chauffe pendant 4 heures à 60°C sous agitation. Après refroidissement, on élimine l'excès d'éthylamine et l'eau sous pression réduite. Après séchage sous pression réduite, on obtient ainsi, avec un rendement de 98 %, 650 mg de 2R,3S-isopropylidènedioxy-4-R-tert-butoxycarbonyl-amino-1-S-éthylaminocarbonylcyclopentane dont la structure est confirmée par le spectre de R.M.N. du proton et dont le pouvoir rotatoire est [α]^{D}₂₀ = 15,0 (c = 1 ; méthanol).

A une solution de 200 mg de 2R,3S-isopropylidènedioxy-4-R-tert-butoxycarbonylamino-1-S-éthylaminocarbonylcyclopentane dans 1,6 cm3 de dichlorométhane anhydre, on ajoute 275 µl d'acide trifluoroacétique. On agite pendant une nuit à une température voisine de -5°C. Le mélange réactionnel est versé dans 4 cm3 de soude aqueuse 2,5N. La couche organique est concentrée sous pression réduite à une température inférieure à 25°C. On obtient ainsi 125 mg d'un produit qui est dissous dans 0,5 cm3 de tétrahydrofurane. A cette solution, on ajoute 70 mg d'acide benzoïque. Après refroidissement de la solution obtenue à une température voisine de 0°C, les cristaux obtenus sont séparés par filtration et lavés au pentane. On obtient ainsi 138 mg de benzoate de 2R,3S-isopropylidènedioxy-4R-amino-1S-éthylaminocarbonylcyclopentane.

### EXEMPLE 6

A une solution de 167 mg de 5R,6S-isopropylidènedioxy-2-(tert.butoxycarbonyl)-2-azabicyclo[2,2,1]heptane-3-one dans 1 cm3 de dichlorométhane, refroidie à 0°C, on ajoute 90 µl d'acide trifluoroacétique.On laisse la température remonter à 23°C en 40 minutes puis on agite pendant 22 heures à cette température. On ajoute à nouveau 90 µl d'acide trifluoroacétique puis agite encore pendant 1 heure à une température de 23°C. Après évaporation sous pression réduite, on obtient 123 mg de 5R,6S-isopropylidènedioxy-2-azabicyclo[2,2,1]heptane-3-one dont la pureté déterminée par chromatographie liquide à haute performance est voisine de 92 % et dont la structure est confirmée par le spectre de R.M.N. du proton.

Une solution de 10 g de 5R,6S-isopropylidènedioxy-2-azabicyclo[2,2,1] heptane-3-one dans 100 cm3 d'une solution aqueuse de triéthylamine à 70 % (en poids) est chauffée à 110°C pendant 20 heures sous pression autogène. Après refroidissement, l'excès de triéthylamine est éliminé sous pression réduite puis on lave au dichlorométhane pour éliminer le produit de départ n'ayant pas réagi. La couche aqueuse est alors concentrée et séchée. On obtient ainsi 10,54 g de 2R,3S-isopropylidènedioxy-4R-amino-1S-éthylaminocarbonylcyclopentane.

## Revendications

1. Dérivés du 2-azabicyclo[2.2.1]heptane 1R de formule générale: 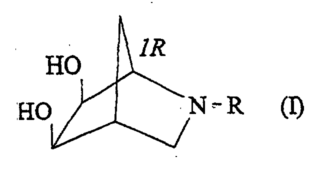 dans laquelle R représente un atome d'hydrogène ou un radical de formule: 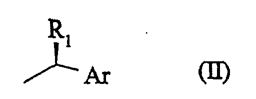 dans laquelle R₁ représente un radical alcoyle contenant 1 à 4 atomes de carbone et Ar représente un radical phényle ou α- ou β-naphtyle éventuellement substitué par un ou plusieurs atomes ou radicaux identiques ou différents choisis parmi les atomes d'halogène et les radicaux alcoyles contenant 1 à 4 atomes de carbone, alcoxy contenant 1 à 4 atomes de carbone ou nitro.

2. Dérivés du 2-azabicyclo[2.2.1]heptane selon la revendication 1 pour lesquels R₁ représente un radical méthyle ou éthyle et Ar représente un radical phényle éventuellement substitué par un ou plusieurs radicaux méthyle ou méthoxy.

3. Dérivé du 2-azabicyclo[2.2.1]heptane selon les revendications 1 ou 2 pour lequel R₁ représente un radical méthyle et Ar représente un radical phényle.

4. Procédé de préparation d'un dérivé selon l'une des revendications 1 à 3 pour lequel R représente un radical de formule générale (II) **caractérisé en ce que** l'on effectue une bis-hydroxylation sur un produit de formule générale : 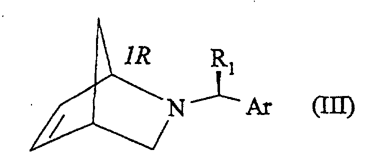 dans laquelle R₁ et Ar sont définis comme dans l'une des revendications 1, 2 ou 3.

5. Procédé selon la revendication 4 **caractérisé en ce que** la bis-hydroxylation est effectuée au moyen de permanganate de potassium ou de tétroxyde d'osmium en opérant en présence de N-méthylmorpholine-oxyde ou de triéthylamine-oxyde ou de ferricyanure de potassium (K₃FeCN₆).

6. Procédé de préparation d'un produit selon la revendication 1 pour lequel R représente un atome d'hydrogène **caractérisé en ce que** l'on traite un produit pour lequel R représente (II) selon l'une des revendications 1 à 3 par l'hydrogène en présence de palladium sur support en opérant dans un solvant organique choisi parmi les alcools aliphatiques contenant 1 à 3 atomes de carbone.

7. Procédé de préparation d'un produit de formule générale : 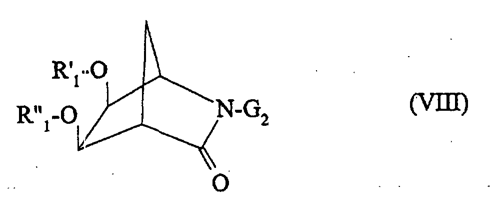 dans laquelle R'₁ et R"₁, identiques ou différents, représentent un reste d'acide organique aliphatique contenant 2 à 4 atomes de carbone, ou un reste benzoyle ou bien R'₁ et R"₁ forment ensemble un radical méthylène dont l'atome de carbone est éventuellement substitué par un ou plusieurs radicaux, identiques ou différents choisis parmi les radicaux alcoyles contenant 1 à 4 atomes de carbone pouvant former ensemble un radical alicyclique contenant 5 ou 6 atomes de carbone ou les radicaux phényles et G₂ représente un groupement protecteur d'un radical amino,
**caractérisé en ce que**:
a) on protège les fonctions hydroxy d'un produit selon l'une des revendications 1, 2 ou 3 pour obtenir un produit de formule générale: 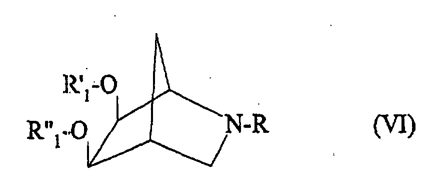 dans laquelle R'₁ et R"₁ sont définis comme ci-dessus et R est défini comme dans l'une des revendications 1, 2 ou 3 dans les conditions habituelles d'estérification ou d'acétalisation,
b) on transforme le produit de formule générale (VI) en produit de formule générale: 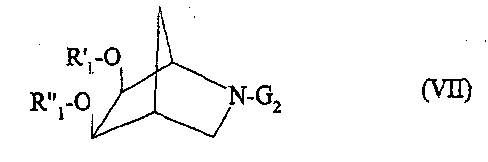 dans laquelle R'₁ et R"₁ sont définis comme précédemment et G₂ représente un groupement protecteur de l'atome d'azote, par, ou bien
i) hydrogénolyse d'un produit de formule générale (VI) dans laquelle R représente un radical de formule générale (II) et action d'un réactif permettant d'introduire un groupement protecteur de l'atome d'azote, l'hydrogénolyse et la protection de l'atome d'azote pouvant être réalisées simultanément, ou bien
ii) action d'un réactif permettant d'introduire un groupement protecteur de l'atome d'azote sur un produit de formule générale (VI) dans laquelle R représente un atome d'hydrogène, puis
c) oxyde le produit de formule générale (VII) en produit de formule générale (VIII).

8. Procédé selon la revendication 7, **caractérisé en ce que** la protection des fonctions hydroxy d'un produit selon l'une des revendications 1 à 3 est effectuée au moyen d'un acide aliphatique contenant 2 à 4 atomes de carbone ou d'un aldéhyde ou d'une cétone éventuellement sous forme d'acétal en présence d'un acide dans un solvant organique inerte à une température entre 50°C et la température de reflux du mélange réactionneL

9. Procédé selon la revendication 7 **caractérisé en ce que** l'hydrogénolyse est effectuée au moyen d'hydrogène en présence de palladium sur charbon et la protection de l'atome d'azote est effectuée dans les conditions habituelles de protection en fonction de la nature du groupement protecteur.

10. Procédé selon la revendication 7 **caractérisé en ce que**, lorsque G₂ représente un radical tert.butoxycarbonyle, l'hydrogénolyse et la protection simultanées sont effectuées en faisant réagir simultanément l'hydrogène en présence de palladium sur charbon et le dicarbonate de di-tert.butyle dans un alcool aliphatique contenant 1 à 3 atomes de carbone à une température comprise entre 0 et 50°C.

11. Procédé selon la revendication 7 **caractérisé en ce que** l'oxydation du produit de formule générale (VII) est effectuée au moyen d'oxyde de ruthénuim (RuO₄) éventuellement généré in situ en présence d'un oxydant.

12. Procédé de préparation d'un produit de formule générale : 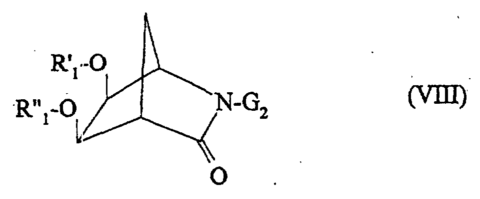 dans laquelle R'₁ et R"₁ et G₂ sont définis comme dans la revendication 7, **caractérisé en ce que** l'on oxyde un produit de formule générale : 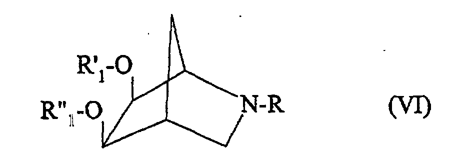 dans laquelle R'₁ et R"₁ sont définis comme dans la revendication 7 et R représente un atome d'hydrogène dans les conditions de la revendication 11 puis protège l'atome d'azote du lactame obtenu dans les conditions de la revendication 10.

13. Un produit de formule générale : 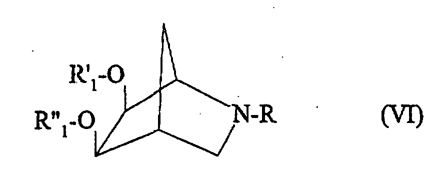 dans laquelle R représente un atome d'hydrogène ou un radical de formule générale (II) tel que défini dans l'une des revendications 1, 2 ou 3 et R'₁ et R"₁, identiques ou différents, représentent un reste d'acide organique aliphatique contenant 2 à 4 atomes de carbone, ou un reste benzoyle ou bien R'₁ et R"₁ forment ensemble un radical méthylène dont l'atome de carbone est éventuellement substitué par un ou plusieurs radicaux, identiques ou différents choisis parmi les radicaux alcoyles contenant 1 à 4 atomes de carbone pouvant former ensemble un radical alicyclique contenant 5 ou 6 atomes de carbone, ou les radicaux phényles.

14. Un produit de formule générale : 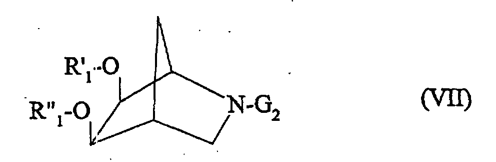 dans laquelle R'₁ et R"₁, identiques ou différents, représentent un reste d'acide organique aliphatique contenant 2 à 4 atomes de carbone, ou un reste benzoyle ou bien R'₁ et R"₁ forment ensemble un radical méthylène dont l'atome de carbone est éventuellement substitué par un ou plusieurs radicaux, identiques ou différents choisis parmi les radicaux alcoyles contenant 1 à 4 atomes de carbone pouvant former ensemble un radical alicyclique contenant 5 ou 6 atomes de carbone, ou les radicaux phényles et G₂ représente un groupement protecteur de la fonction amino.

## Claims

1. (1R)-2-Azabicyclo[2.2.1]heptane derivative of general formula: 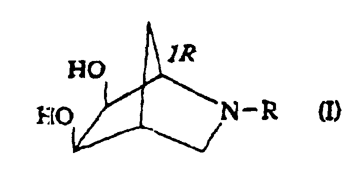 in which R represents a hydrogen atom or a radical of the formula: 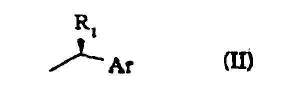 in which R₁ represents an alkyl radical containing 1 to 4 carbon atoms and Ar represents a phenyl or α- or β-naphthyl radical optionally substituted by one or more identical or different atoms or radicals chosen from halogen atoms and alkyl radicals containing 1 to 4 carbon atoms, alkoxy radicals containing 1 to 4 carbon atoms ,r the nitro radical.

2. 2-Azabicyclo[2.2.1]heptane derivative according to Claim 1 for which R₁ represents a methyl or ethyl radical and Ar represents a phenyl radical optionally substituted by one or more methyl or methoxy radicals.

3. 2-Azabicyclo[2.2.1]heptane derivative according to Claim 1 or 2 for which R₁ represents a methyl radical and Ar represents a phenyl radical.

4. Process for the preparation of a derivative according to one of Claims 1 to 3 for which R represents a radical of general formula (II), **characterized in that** a bishydroxylation is carried out on a product of general formula: 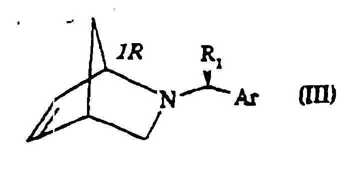 in which R₁ and Ar are defined as in one of Claims 1, 2 or 3.

5. Process according to Claim 4, **characterized in that** the bishydroxylation is carried out by means of potassium permanganate or of osmium tetroxide, the reaction being carried out in the presence of N-methylmorpholine oxide or of triethylamine oxide or of potassium ferricyanide (K₃FeCN₆).

6. Process for the preparation of a product according to Claim 1 for which R represents a hydrogen atom, **characterized in that** a product for which R represents (II) according to one of Claims 1 to 3 is treated with hydrogen in the presence of palladium on a support, the reaction being carried out in an organic solvent chosen from aliphatic alcohols containing 1 to 3 carbon atoms.

7. Process for the preparation of a product of genera] formula: 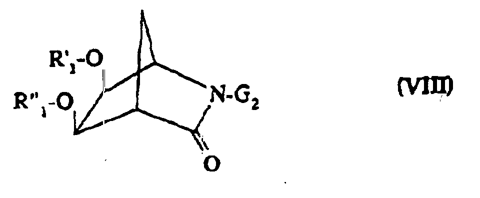 in which R'₁ and R"₁, which are identical or different, represent an aliphatic organic acid residue containing 2 to 4 carbon atoms or a benzoyl residue, or else R'₁ and R"₁ together form a methylene radical, the carbon atom of which is optionally substituted by one or more identical or different radicals chosen from alkyl radicals containing 1 to 4 carbon atoms, which can together form an alicyclic radical containing 5 or 6 carbon atoms, or phenyl radicals, and G₂ represents a protecting group for an amino radical,
**characterized in that**:
a) the hydroxyl functional groups of a product according to one of Claims 1, 2 or 3 are protected under the usual esterification or acetalization conditions in order to obtain a product of general formula: 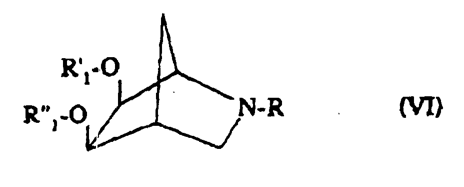 in which R'₁ and R"₁ are defined as above and R is definec as in one of Claims 1, 2 or 3,
b) the product of general formula (VI) is converted into the product of general formula: 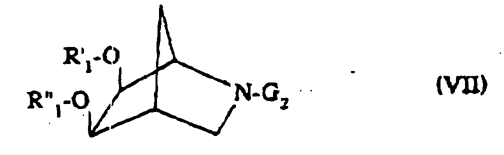 in which R'₁ and R"₁ are defined as above and G₂ represents a protecting group for the nitrogen atom, by either
i) hydrogenolysis of a product of general formula (VI) in which R represents a radical of general formula (II) and reaction with a reagent which allows a protecting group for the nitrogen atom to be introduced, it being possible for the hydrogenolysis and the protection of the nitrogen atom to be carried out simultaneously, or else
ii) reaction of a product of general formula (VI) in which R represents a hydrogen atom with a reagent which allows a protecting group for the nitrogen atom to be introduced, then
c) the product of general formula (VII) is oxidized to the product of general formula (VIII).

8. Process according to Claim 7, **characterized in that** the protection of the hydroxyl functional groups of a product according to one of Claims 1 to 3 is carried out by means of an aliphatic acid containing 2 to 4 carbon atoms or of an aldehyde or of a ketone, optionally in acetal form, in the presence of an acid in an inert organic solvent at a temperature between 50°C and the reflux temperature of the reaction mixture.

9. Process according to Claim 7, **characterized in that** hydrogenolysis is carried out by means of hydrogen in the presence of palladium-on-carbon and the protection of the nitrogen atom is carried out under the usual conditions for protection, depending on the nature of the protecting group.

10. Process according to Claim 7, **characterized in that**, when G₂ represents a tert-butoxycarbonyl radical, the simultaneous hydrogenolysis and protection are carried out by simultaneously reacting hydrogen in the presence of palladium-on-carbon and di-tert-butyl dicarbonate in an aliphatic alcohol containing 1 to 3 carbon atoms at a temperature of between 0 and 50°C.

11. Process according to Claim 7, **characterized in that** the oxidation of the product of general formula (VII) is carried out by means of ruthenium oxide (RuO₄), optionally generated in situ in the presence of an oxidizing agent.

12. Process for the preparation of a product of general formula: 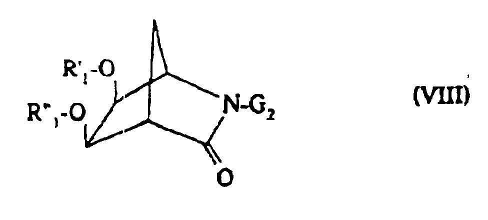 in which R'₁ and R"₁ and G₂ are defined as in Claim 7, **characterized in that** a product of general formula: 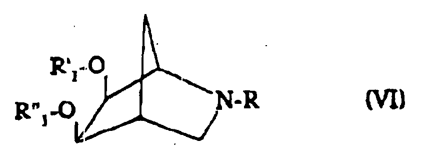 in which R'₁ and R"₁ are defined as in Claim 7 and R represents a hydrogen atom, is oxidized under the conditions of Claim 11 and then the nitrogen atom of the lactam obtained is protected under the conditions of Claim 10.

13. A product of general formula: 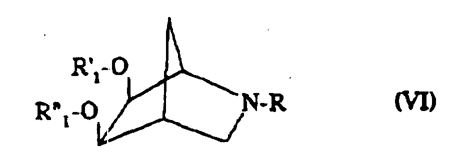 in which R represents a hydrogen atom or a radical of general formula (II) as defined in one of Claims 1, 2 or 3 and R'₁ and R"₁, which are identical or different, represent an aliphatic organic acid residue containing 2 to 4 carbon atoms or a benzoyl residue, or else R'₁ and R" together form a methylene radical, the carbon atom of which is optionally substituted by one or more identical or different radicals chosen from alkyl radicals containing 1 to 4 carbon atoms, which can together form an alicyclic radical containing 5 or 6 carbon atoms, or phenyl radicals.

14. A product of general formula: 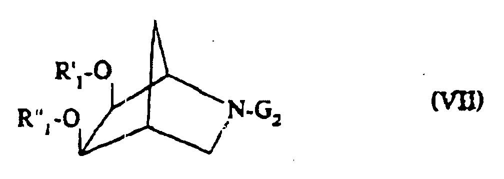 in which R'₁ and R"₁, which are identical or different, represent an aliphatic organic acid residue containing 2 to 4 carbon atoms or a benzoyl residue, or else R'₁ and R"₁ together form a methylene radical, the carbon atom of which is optionally substituted by one or more identical or different radicals chosen from alkyl radicals containing 1 to 4 carbon atoms, which can together form an alicyclic radical containing 5 or 6 carbon atoms, or phenyl radicals, and G₂ represents a protecting group for the amino functional group.

## Patentansprüche

1. 1R-2-Azabicyclo[2.2.1]heptan-Derivate der allgemeinen Formel: 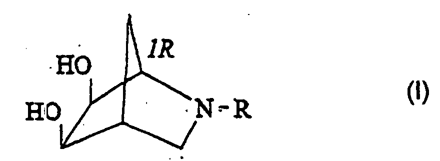 in der R ein Wasserstoffatom oder einen Rest der Formel: 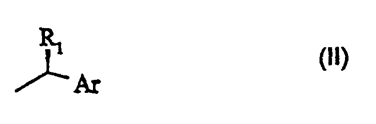 darstellt, in der R₁ einen Alkyllrest mit 1 bis 4 Kohlenstoffatomen darstellt und Ar einen Phenyl- oder α- oder β-Naphthylrest darstellt, der gegebenenfalls mit einem oder mehreren, gleichen oder verschiedenen Atomen oder Resten substituiert ist, die unter den Halogenatomen und den Alkylresten mit 1 bis 4 Kohlenstoffatomen, Alkoxyresten mit 1 bis 4 Kohlenstoffatomen oder dem Nitrorest ausgewählt sind.

2. 2-Azabicyclo[2.2.1]heptan-Derivate gemäß Anspruch 1, für die R₁ einen Methyl- oder Ethylrest darstellt und Ar einen Phenylrest darstellt, der gegebenenfalls mit einem oder mehreren Methyl- oder Methoxyresten substituiert ist

3. 2-Azabicyclo[2.2.1]heptan-Derivat gemäß den Ansprüchen 1 oder 2, für das R₁ einen Methylrest darstellt und Ar einen Phenylrest darstellt.

4. Verfahren zur Herstellung eines Derivats gemäß einem der Ansprüche 1 bis 3, für das R einen Rest der allgemeinen Formel (II) darstellt, **dadurch gekennzeichnet**, dass man eine Bishydroxylierung an einem Produkt der allgemeinen Formel: 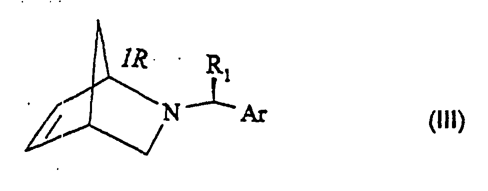 in der R₁ und Ar wie in einem der Ansprüche 1, 2 oder 3 definiert sind, ausführt.

5. Verfahren gemäß Anspruch 4, **dadurch gekennzeichnet**, dass die Bishydroxylierung mittels Kaliumpermanganat oder Osmiumtetroxid ausgeführt wird, indem man in Gegenwart von N-Methylmorpholin-oxid oder Triethylamin-oxid oder Kaliumferricyanid (K₃FeCN₆) arbeitet.

6. Verfahren zur Herstellung eines Produkts gemäß Anspruch 1, für das R ein Wasserstoffatom darstellt, **dadurch gekennzeichnet**, dass man ein Produkt, für das R gemäß einem der Ansprüche 1 bis 3 (II) darstellt, mit Wasserstoff in Gegenwart von Palladium auf einem Träger behandelt, indem man in einem organischen Lösungsmittel arbeitet, das unter den aliphatischen Alkoholen mit 1 bis 3 Kohlenstoffatomen ausgewählt ist.

7. Verfahren zur Herstellung eines Produkts der allgemeinen Formel: 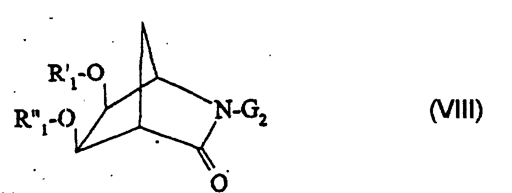 in der R'₁ und R"₁, die gleich oder verschieden sind, einen aliphatischen organischen Säurerest mit 2 bis 4 Kohlenstoffatomen oder einen Benzoylrest darstellen oder R'₁ und R"₁ zusammen einen Methylenrest bilden, dessen Kohlenstoffatom gegebenenfalls mit einem oder mehreren, gleichen oder verschiedenen Resten substituiert ist, die ausgewählt sind unter den Alkylresten mit 1 bis 4 Kohlenstoffatomen, die zusammen einen alicyclischen Rest mit 5 oder 6 Kohlenstoffatomen bilden können, oder den Phenylresten, und G₂ eine Schutzgruppe für einen Aminorest darstellt, **dadurch gekennzeichnet**, dass:
a) man die Hydroxyfunktionen eines Produkts gemäß einem der Ansprüche 1, 2 oder 3 schützt, um ein Produkt der allgemeinen Formel: 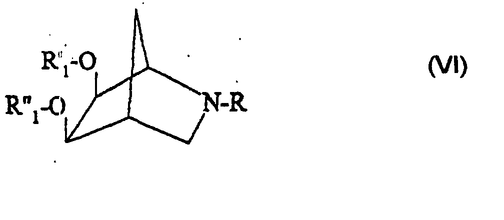 in der R'₁ und R"₁ wie oben definiert sind und R wie in einem der Ansprüche 1, 2 oder 3 definiert ist, unter den üblichen Bedingungen einer Veresterung oder Acetalisierung zu erhalten,
b) man das Produkt der allgemeinen Formel (VI) umwandelt in ein Produkt der allgemeinen Formel: 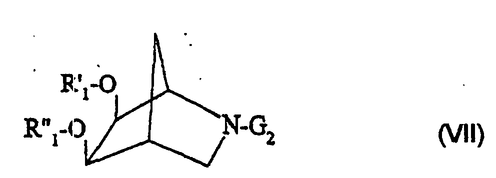 in der R'₁ und R"₁ wie zuvor definiert sind und G₂ eine Schutzgruppe für das Stickstoffatom darstellt, entweder durch
i) Hydrogenolyse eines Produkts der allgemeinen Formel (VI), in der R einen Rest der allgemeinen Formel (II) darstellt, und Einwirkung eines Reagenzes, das es ermöglicht, eine Schutzgruppe für das Stickstoffatom einzuführen, wobei die Hydrogenolyse und das Schützen des Stickstoffatoms gleichzeitig durchgeführt werden können, oder durch
ii) Einwirkung eines Reagenzes, das es ermöglicht, eine Schutzgruppe für das Stickstoffatom einzuführen, auf ein Produkt der allgemeinen Formel (VI), in der R ein Wasserstoffatom darstellt, dann
c) das Produkt der allgemeinen Formel (VII) zu dem Produkt der allgemeinen Formel (VIII) oxidiert.

8. Verfahren gemäß Anspruch 7, **dadurch gekennzeichnet**, dass das Schützen der Hydroxyfunktionen eines Produkts gemäß einem der Ansprüche 1 bis 3 mittels einer aliphatischen Säure mit 2 bis 4 Kohlenstoffatomen oder eines Aldehyds oder eines Ketons gegebenenfalls in Acetatform in Gegenwart einer Säure in einem inerten organischen Lösungsmittel bei einer Temperatur zwischen 50 °C und der Rückflusstemperatur des Reaktionsgemischs ausgeführt wird.

9. Verfahren gemäß Anspruch 7, **dadurch gekennzeichnet**, dass die Hydrogenolyse mittels Wasserstoff in Gegenwart von Palladium auf Kohle ausgeführt wird und das Schützen des Stickstoffatoms unter den üblichen Bedingungen für das Einführen einer Schutzgruppe in Abhängigkeit von der Art der Schutzgruppe ausgeführt wird.

10. Verfahren gemäß Anspruch 7, **dadurch gekennzeichnet**, dass, wenn G₂ einen tert.-Butoxycarbonylrest darstellt, die gleichzeitige Hydrogenolyse und Einführung der Schutzgruppen ausgeführt werden, indem man den Wasserstoff in Gegenwart von Palladium auf Kohle und das Di-tert.-butyl-dicarbonat gleichzeitig in einem aliphatischen Alkohol mit 1 bis 3 Kohlenstoffatomen bei einer Temperatur zwischen 0 und 50 °C umsetzt.

11. Verfahren gemäß Anspruch 7, **dadurch gekennzeichnet**, dass die Oxidation des Produkts der allgemeinen Formel (VII) mittels Rutheniumoxid (RuO₄) ausgeführt wird, das gegebenenfalls in situ in Gegenwart eines Oxidationsmittels erzeugt wird.

12. Verfahren zur Herstellung eines Produkts der allgemeinen Formel: 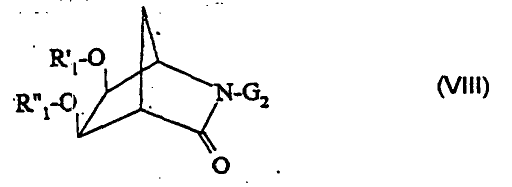 in der R'₁ und R"₁ und G₂ wie in Anspruch 7 definiert sind, **dadurch gekennzeichnet**, dass man ein Produkt der allgemeinen Formel: 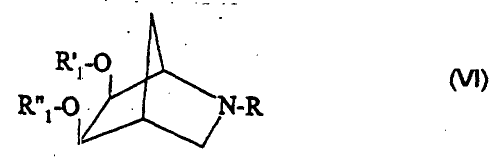 in der R'₁ und R"₁ wie in Anspruch 7 definiert sind und R ein Wasserstoffatom darstellt, unter den Bedingungen des Anspruchs 11 oxidiert, dann das Stickstoffatom des erhaltenen Lactams unter den Bedingungen des Anspruchs 10 schützt.

13. Ein Produkt der allgemeinen Formel: 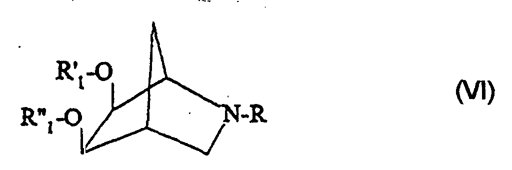 in der R ein Wasserstoffatom oder einen Rest der allgemeinen Formel (II), wie in einem der Ansprüche 1, 2 oder 3 definiert, darstellt und R'₁ und R"₁, die gleich oder verschieden sind, einen aliphatischen organischen Säurerest mit 2 bis 4 Kohlenstoffatomen oder einen Benzoylrest darstellen oder R'₁ und R"₁ zusammen einen Methylenrest bilden, dessen Kohlenstoffatom gegebenenfalls mit einem oder mehreren, gleichen oder verschiedenen Resten substituiert ist, die ausgewählt sind unter den Alkylresten mit 1 bis 4 Kohlenstoffatomen, die zusammen einen alicyclischen Rest mit 5 oder 6 Kohlenstoffatomen bilden können, oder den Phenylresten.

14. Ein Produkt der allgemeinen Formel: 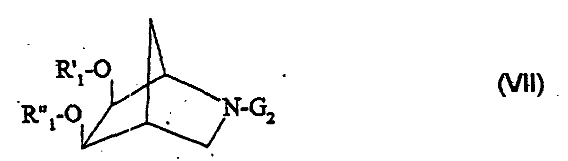 in der R'₁ und R"₁, die gleich oder verschieden sind, einen aliphatischen organischen Säurerest mit 2 bis 4 Kohlenstoffatomen oder einen Benzoylrest darstellen oder R'₁ und R"₁ zusammen einen Methylenrest bilden, dessen Kohlenstoffatom gegebenenfalls mit einem oder mehreren, gleichen oder verschiedenen Resten substituiert ist, die ausgewählt sind unter den Alkylresten mit 1 bis 4 Kohlenstoffatomen, die zusammen einen alicyclischen Rest mit 5 oder 6 Kohlenstoffatomen bilden können, oder den Phenylresten, und G₂ eine Schutzgruppe für die Aminofunktion darstellt.
